# EUROPÄISCHE PATENTANMELDUNG

(11) **EP 1 074 313 A2**
(43) Veröffentlichungstag der Anmeldung: **07.02.2001**
(21) Anmeldenummer: 00810653.6
(22) Anmeldetag: 21.07.2000
(51) Int. Cl.: B09B 3/00, C05F 9/02, B65G 65/40

(54) **Vergärungsanlage mit Vorrichtung zur Materialbeschickung**

(30) Priorität: 28.07.1999 CH 140899
(71) Anmelder: Walter Schmid AG, 8152 Glattbrugg (CH)
(72) Erfinder: Kern, Daniel, 8302 Kloten (CH)
(74) Vertreter: Patentanwaltsbüro Feldmann AG

(57) **Zusammenfassung**

Ein Fermenter (1) einer kontinuierlich oder quasi kontinuierlich arbeitenden Fermentieranlage weist eine Annahmeeinheit (30) auf, von der das Frischgut entnommen und bei Bedarf zerkleinert in ein Zwischenlager (20) abgegeben werden kann. Das erfindungsgemässe Zwischenlager umfasst einen Laderaum (21), welcher als Muldenkonstruktion (22) gestaltet ist. Der Boden des Laderaumes (21) ist als Förderboden (23) gestaltet, der durch einen Antrieb (25) bewegbar ist. In der Austragsrichtung am Ende ist eine Dosier- und Austragseinheit (24) angeordnet. Mittels der Dosier- und Austragseinheit (24) und dem Förderboden (23) kann das zerkleinerte Frischgut aus dem Laderaum (21) kontinuierlich oder quasi kontinuierlich dosiert auf ein etwa horizontal verlaufendes Förderband (27) abgegeben werden. Mittels einem zweiten Förderband (28) lässt sich das Material weiter in eine Speiseeinheit (3) fördern, von wo es über die vorzugsweise beheizte Speiseleitung (2) in den Fermenter (1) eingetragen werden kann.

## Beschreibung

Die vorliegende Erfindung betrifft eine kontinuierlich oder quasi kontinuierlich arbeitende Anlage zum Fermentieren beziehungsweise Vergären von biogenen Abfällen, die mindestens je eine Annahmeeinheit, ein Zwischenlager, eine Speiseeinheit und einen Fermenter umfasst.

Anlagen der eingangs genannten Art haben sich ausserordentlich bewährt. Neben einer ökologischen Abfallbewirtschaftung werden mit solchen Anlagen bedeutende Mengen von Biogas erzeugt, die einerseits zum Betrieb einer BHKW-Anlage eingesetzt werden können und deren Energieüberschuss andererseits entweder in ein Erdgasnetz oder für Antriebszwecke von Motorfahrzeugen Verwendung finden kann.

Solche Anlagen stehen immer auch in Konkurrenz mit anderen Abfallbewirtschaftungsmethoden. So stehen beispielsweise solche Anlagen in Konkurrenz mit Kehrichtsverbrennungsanlagen. Kehrichtsverbrennungsanlagen sind zwar weder ökologisch noch ökonomisch, doch eben vielfach bereits bestehend. Sie wurden konzipiert zu einer Zeit, als die getrennte Abfallsammlung in verschiedene Fraktionen kaum üblich war. Entsprechend sind diese Anlagen für grosse Kapazitäten konzipiert und besitzen heute wegen der getrennten Abfallbewirtschaftung Überkapazitäten. Entsprechend nehmen Kehrichtsverbrennungsanlagen heute Haushaltmüll zu extrem tiefen Preisen entgegen, was der ökologisch vernünftigen getrennten Abfallsammlung und Entsorgung entgegen wirkt.

Zum weiteren stehen die eingangs genannten Anlagen zum Fermentieren biogener Abfälle auch mit den erheblich einfacheren Abfallrotten in Konkurrenz. Die biogenen Abfälle werden hier offen, also aerob, abgebaut. Anlagemässig brauchen solche Roten nur einen geringen Aufwand, müssen doch lediglich die verrottenden biogenen Abfälle langsam umgeschichtet werden, was mit einfachsten, auf dem Markt erhältlichen Maschinen geschehen kann. Die hierbei freigesetzten Biogase entweichen ungenutzt und belasten die Atmosphäre. Den grössten Kostenpunkt stellt das relativ grosse Areal dar, welches für solche Anlagen benötigt wird. Die auftretenden Geruchsemissionen machen es in der Regel unmöglich solche Anlagen direkt in Siedlungsgebieten oder an Siedlungsgebiete grenzend zu erstellen und zu betreiben.

Viel aufwendiger als die Verrottungsanlagen sind die Anlagen, In denen biogene Abfälle fermentiert beziehungsweise vergärt werden. Die Vergärung erfolgt hierbei anaerob, was relativ grosse, geschlossene Fermenter erfordert. Gemäss den heute bekannten Konzepten werden sämtliche Anlagebereiche als stationäre Baueinheiten erstellt, die erhebliche Gebäudeteile und viele Infrastrukturen benötigen, was solche Anlagen wiederum kostspielig machen.

Bei den erwähnten, von der Anmelderin weltweit erstellten Anlagen wird bis heute so gearbeitet, dass die biogenen Abfälle in einen Annahmebunker gefüllt wurden und von dort über einen Dosierer unter Zwischenschaltung einer Sortieranlage in einen Zerkleinerer gelangen, von dem aus über feste Schnecken- oder Bandförderer das zerkleinerte Biogut in ein Zwischenlager transportiert wird. Aus dem Zwischenlager wird das Material direkt oder indirekt entnommen und via eines Wärmetauschers dem Fermenter oder Gärreaktor zugeführt. Die Biogut-Zwischenlager sind relativ gross ausgelegt, wobei, um die Grundfläche klein zu halten, diese Zwischenlager meist eine erhebliche Höhe aufwiesen. Entsprechend entsteht im als Bunker gestalteten Zwischenlager auch ein relativ hoher spezifischer Druck, so dass bereits in diesem Bereich unerwünschterweise Press- oder Abwasser entsteht, welches biologisch beladen ist und zu hygienischen Problemen führen kann. Auch kann bereits im Zwischenlager eine unerwünschte Vergärung beginnen, die zu einem ebenfalls unerwünschten Biogasverlust führt.

Wesentlicher als dieser nicht sehr bedeutende Energieverlust sind jedoch die Kosten des Bunkerbaus sowie die kostspieligen und auch Funktionsstörungen unterliegenden Entnahmevorrichtungen und Förderstrecken, die entweder als Förderbänder oder als Schneckenförderer ausgestaltet sind. Dies verlangt verschiedene Antriebsmittel, Schieber, Schleusen, Pressen und Pumpen, die entsprechende Kosten verursachen, der Abnutzung unterliegen und wegen der im Gebäude fest angeordneten Unterbringung auch nur bedingt unterhaltsfreundlich sind.

Es ist somit die Aufgabe der vorliegenden Erfindung, eine Anlage zu schaffen, bei der die biogenen Abfälle einfacher zwischengelagert und dosiert der Speisung des Fermenters zugeführt werden können und die mit geringeren Investitionen realisiert werden kann.

Diese Aufgabe löst eine Anlage zum Fermentieren beziehungsweise Vergären von biogenen Abfällen, welche die Merkmale des Patentanspruches 1 aufweist.

Dank dieser Anlage fällt ein als Gebäudeteil realisierter Zwischenbunker weg. Statt eines Zwischenbunkers ist nun ein Zwischenlager als Laderaum mit Förderboden und Dosier- und Austragseinheit vorhanden, so dass nunmehr die Zufuhr zum Fermenter praktisch als Materialpufferstrecke konzipiert ist. Damit entfallen auch die Kosten für Umwälz- oder Umschichtsysteme die verhindern sollen, dass Material über längere Zeit in einem Zwischenbunker liegen bleibt.

Besonders kostengünstig lässt sich ein solcher Laderaum als offene Muldenkonstruktion, vorzugsweise aus Metall und/oder korrosionsbeständigem Kunststoff, herstellen. Bei Anlagen, in denen das angelieferte Biomaterial zu grob zum Vergären ist, muss vorteilhafterweise vorgängig zerkleinert werden. Hier lässt sich der Laderaum besonders vorteilhaft beschicken, wenn man einen Zerkleinerer mobil gestaltet. Ein solcher Zerkleinerer kann fahrbar oder schwenkbar gebaut werden. Der Zerkleinerer kann mit einem Aufnahmetrichter sowie gegebenenfalls mit einem Sammelbehälter versehen sein. Dank der mobilen Anordnung des Zerkleinerers kann die Beschickung des Laderaumes immer hinten an das bereits vorhandene Material anschliessend erfolgen und ist nicht mehr rein ortsgebunden. Damit kann zum Beispiel erreicht werden, dass das Frischgut in gleicher zeitlicher Folge zum Fermenter gelangt, wie es in den Laderaum eingefüllt wird.

Weitere vorteilhafte Ausgestaltungsformen des Erfindungsgegenstandes gehen aus den weiteren abhängigen Ansprüchen hervor, deren Bedeutung in der nachfolgenden Beschreibung erläutert ist.

In der beiliegenden Zeichnung ist eine bevorzugte Ausführungsform des Erfindungsgegenstandes dargestellt und nachfolgend erläutert.
- Figur 1: zeigt eine Gesamtansicht der erfindungsgemässen Anlage in vereinfachter Darstellung und unter Weglassung der infrastrukturellen Einheiten und der Anlagenteile, die mit der Gasgewinnung, Lagerung und Energieumwandlung für interne und externe Zwecke zusammenhängen.
- Figur 2: stellt einen Vertikalschnitt durch die Anlage gemäss der Figur 1 entlang der Linie A-A dar.
- Figur 3: zeigt insbesondere die infrastrukturellen Massnahmen im Bereich der Annahme der biogenen Abfälle und der Weiterbehandlung des vergorenen Gutes.

Die anlagemässig grösste Baueinheit stellt selbstverständlich der Fermenter oder Gärreaktor 1 dar. Dieser wird einseitig über eine vorzugsweise beheizte Speiseleitung 2 im Propfstrombetrieb kontinuierlich oder quasi kontinuierlich mit frischen biogenen Abfällen gespiesen. Dies erfolgt mittels einer Speiseeinheit 3. Die Speiseeinheit 3 ist zusätzlich über eine Ansaugleitung 4 mit dem Fermenter oder Garreaktor verbunden. Die Speiseeinheit 3 ist so gestaltet, dass sie sowohl in der einen als auch in der anderen Richtung zu fördern vermag. Dies kann beispielsweise mittels einem bidirektionalen Pumpsystem erfolgen. Über die Leitung 4 kann somit nahe dem Austrittsbereich vergorenes Gut dem Fermenter 1 entnommen werden und in die Speiseleitung 2 zur Impfung des Frischgutes eingespeist werden. Entsprechend ist die Speiseeinheit 3 mit Antriebsmitteln 5 versehen. Sie weist ferner eine Beschickungsöffnung 6 auf, welche von einem Sieb abgedeckt sein kann und über die das gesiebte Frischgut in die bidirektional wirkende Speiseeinheit 3 gelangt. Der Zerkleinerer 7 ist in dieser Figur nicht erkennbar. Das im Pfropfstrombetrieb durch den Fermenter 1 geförderte Material gelangt über eine Austragsschnecke 8 nach aussen. Das ausgetragene, vergorene Material gelangt noch auf Nachrotten, bevor es als hochwertiger Kompost weiterverwendet wird.

Zur Regulierung des Verfahrens kann Wasser über Leitungen und ein Wasserbeimischungsventil 9 dem Frischgut nach Bedarf, vorzugsweise im Bereich der Speiseeinheit 3, beigegeben werden.

Das Frischgut, das von einer hier nicht erkennbaren Frischgutannahme kommt, wird von dort auf einen hier ebenfalls nicht sichtbaren Zerkleinerer gegeben. Dieser Zerkleinerer kann mobil gestaltet sein, so dass das zerkleinerte Frischgut direkt in das erfindungsgemässe Zwischenlager 20 abgegeben werden kann. Der Zerkleinerer kann das zerkleinerte Frischgut auch an einer anderen Stelle auswerfen, so dass es in einem weiteren Schritt, zum Beispiel mittels eines Kranes oder Greifers dem Zwischenlager 20 zugeführt werden kann. Dieses Zwischenlager 20 besteht aus einem Laderaum 21, der aus einer oben offenen, langgestreckten Mulde 22, zum Beispiel einer Metallmulde, besteht. Der Boden des Laderaumes 21 ist als Förderboden 23 realisiert. Der Förderboden 23 kann wie dies in der Technik beispielsweise für Ladewagen im Agrarbereich bekannt ist, als Kratzboden konstruiert sein. Auch die Realisierung des Förderbodens als Endlos-Förderband, oder als Moving- oder Runnig-Floor sind bekannte Ausgestaltungsmöglichkeiten. Die Förderrichtung des Förderbodens 23 ist in der Zeichnung mit einem Pfeil F angegeben. Am Ende des Laderaumes 21 beziehungsweise der Mulde 22 ist eine Dosier- und Austragseinheit 24 dargestellt. Die Dosier- und Austragseinheit 24 kann ebenfalls gleich gestaltet sein wie bei einem Ladewagen. Die Dosier- und Austragseinheit 24 braucht aber nicht zwingend ein aktiv förderndes Element zu sein. Die Dosier- und Austragseinheit 24 kann im einfachsten Fall ein Rückhalteelement sein. Ein solches Rückhalteelement entspricht in etwa einer Stau- oder Schleusenplatte. Diese Stau- oder Schleusenplatte lässt sich in der Höhe und/oder in der Distanz relativ zum Förderboden verstellen. Damit lässt sich die Austragsmenge des Frischgutes, welches auf dem Förderboden liegt und in Richtung zur Dosier- und Austragseinheit 24 befördert wird, dosiert abgeben.

Verwendet man statt des passiven Rückhalteelementes eine aktive Dosier- und Austragseinheit, so lässt sich dadurch die dosierte kontinuierliche Abgabe des Frischgutes aus dem Zwischenlager 20 besser kontrollieren. Eine solche Dosier-und Austragseinheit 24 kann aber durchaus auch als umlaufender Kettenkratzer gestaltet sein. Dieser Kettenkratzer fördert dann das Material am Ende des Förderbodens 23 durch einen hier nicht erkennbaren Austragsspalt nach unten auf eine Förderstrecke, die schliesslich zur Speiseeinheit 3 führt.

In der Zeichnung ist hier der Antrieb des Förderbodens erkennbar, welcher mit der Bezugszahl 25 bezeichnet ist. Im gleichen Bereich befindet sich praktisch in Blickrichtung senkrecht darüber oder darunter ein Antrieb 26, welcher die Dosier- und Austragseinheit 24 antreibt. Wie bereits erwähnt, gelangt das ausgetragene Material auf eine Förderstrecke, die zur Speiseeinheit 3 führt. Diese Förderstrecke besteht einerseits aus einem praktisch horizontal verlaufenden Förderband 27 und einem schräg nach oben zur Beschickungsöffnung 6 der Speiseeinheit 3 aufsteigenden Förderband 28.

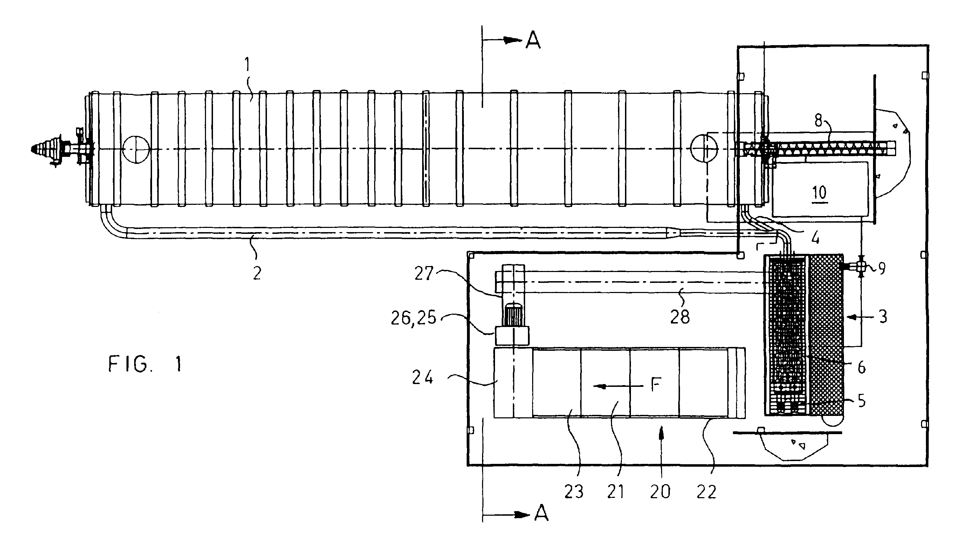

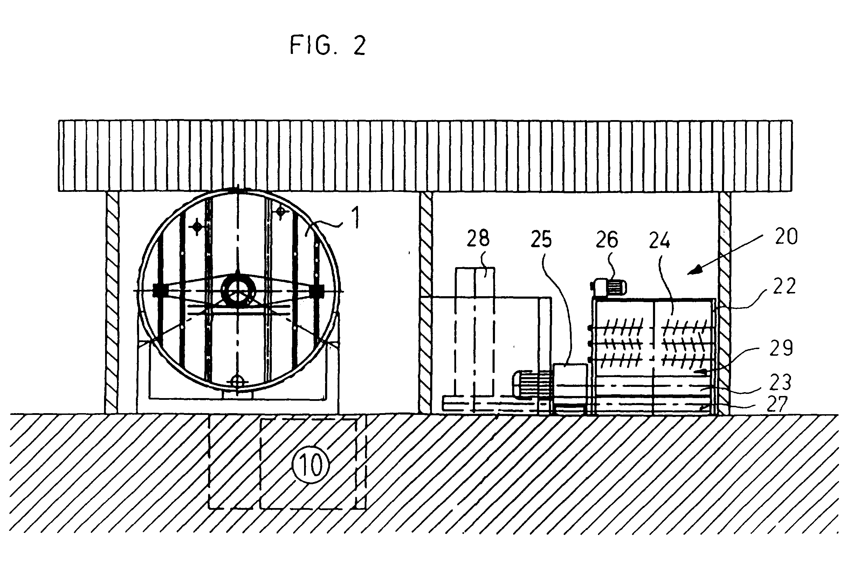

In der Figur 2 ist ein schematisch dargestellter Schnitt durch die Anlage nach Figur 1 entlang der Linie A-A gezeichnet. Hier ist der Fermenter beziehungsweise der Gärreaktor 1 im Schnitt dargestellt, und man erkennt dessen zylindrischen Querschnitt. Allerdings spielt für die Erfindung die Querschnittsform des Gärreaktors keine Rolle. So können selbstverständlich auch Fermenter mit U-förmigen oder rechteckigen Querschnittsformen verwendet werden. Es ist auch unwesentlich, ob der Fermenter als Betonfermenter oder wie hier dargestellt als Stahlkonstruktion gefertigt ist. Rechts neben dem Fermenter in einem getrennten Raum ist das Zwischenlager 20 rechts erkennbar. Man sieht die beiden Seitenwände der Mulde 22 sowie die Dosier- und Austragseinheit 24 und den Förderboden 23. Der Raum zwischen der Dosier- und Austragseinheit 24 und dem Förderboden 23 ist der früher bereits erwähnte Austragsspalt 29. Unterhalb des Förderbodens 23 verläuft das erwähnte horizontal angeordnete Förderband 27, welches das dosiert abgegebene Frischgut weiterfördert und auf das schräg nach oben verlaufende Förderband 28 abgibt, welches zur Speiseeinheit führt.

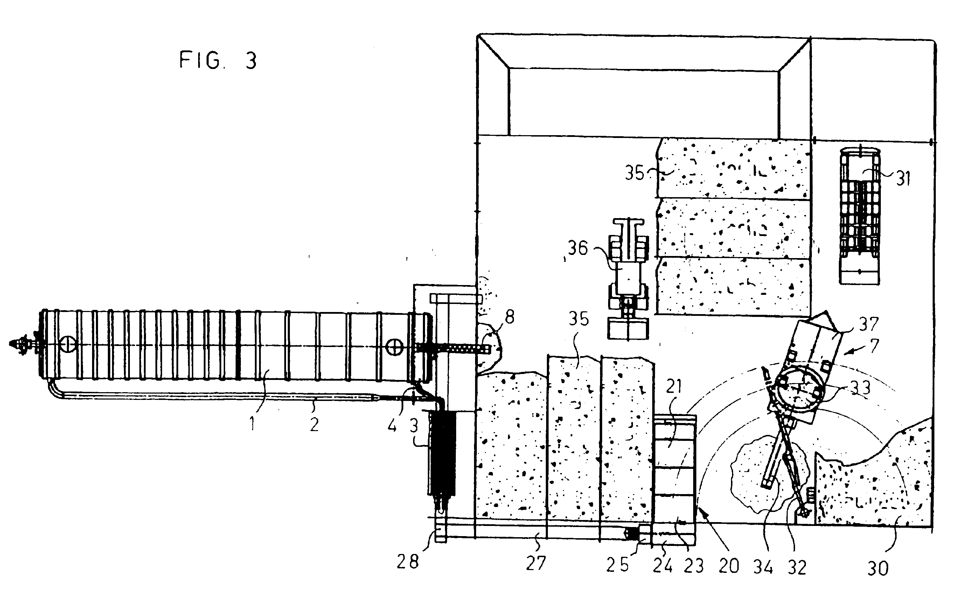

In der Figur 3 ist eine etwas anders dargestellte Anlage gezeichnet. Unverändert ist auch hier ein Fermenter oder Gärreaktor 1 vorhanden, welcher von einer vorzugsweise beheizten Speiseleitung gespeist wird. Die bidirektional arbeitende Speiseeinheit 3 ist auch hier wiederum über eine Ansaugleitung 4 mit dem Fermenter 1 verbunden. In dieser Figur ist aber zusätzlich die Annahmeeinheit 30 wiedergegeben, die in der dargestellten Ausführungsform als Annahmeraum 30 dargestellt ist. Ein Lastwagen 31 kann das herangefahrene Frischgut im Annahmeraum 30 deponieren. Als erfinderische Besonderheit ist hier ein mobiler Zerkleinerer 7 dargestellt. Über einen Kran 32 ist der mobile Zerkleinerer 7 beschickbar und mittels des Kranes kann das zerkleinerte Material auch dem Laderaum zugeführt werden. Der Zerkleinerer umfasst einen relativ grossen Fülltrichter 33, der wie bereits erwähnt mittels Kran 32 beschickt werden kann. Der mobile Zerkleinerer ist so angeordnet und bewegbar, dass er Frischgut aus dem Annahmeraum 30 zerkleinern und danach im zerkleinerten Zustand in den Laderaum 21 oder an einer anderen Stelle im Schwenkbereich des Kranes 32 abgeben kann. Der Zerkleinerer 7 weist entsprechende Messerwalzen und/oder Mahlwalzen auf, um das Frischgut in die gewünschte Einheitsgrösse zu zerkleinern. Vorsichtshalber kann über dem Trichter 33 ein Sieb vorhanden sein, um gewisse, zu grosse Elemente abzutrennen. Das zerkleinerte Material wird mittels der Austragseinheit 34 abgegeben, hier können mittels eines Magnetabscheiders unerwünschte Metallteile entfernt werden. In der Figur ist mit 37 die Antriebseinheit des Zerkleinerers dargestellt.
Der Einsatz eines Zerkleinerers ist verständlicherweise abhängig von der Beschaffenheit des zur Gäranlage angelieferten Bioabfalles. Falls das Material schon vorgängig zerkleinert wurde, zum Beispiel vor dem Transport, oder falls das Material keiner Zerkleinerung bedarf, so ist auch kein Zerkleinerer in der Vergärungsanlage nötig. Die in den Ausführungsbeispielen gezeigten mobilen Zerkleinerer haben den Vorteil, dass sie in solchen Fällen sehr einfach aus der Anlage entfernt und zum Beispiel anderweitig eingesetzt werden können. Wird das Frischgut in Containern angeliefert, so kann die Annahmeeinheit von einem Standplatz für einen solchen Container gebildet werden. Liegt das im Container angelieferte Material bereits in einer Partikelgrösse vor, die ein Einspeisen in den Fermenter erlaubt, so kann ein solcher Container vorteilhafterweise direkt an den Laderaum 21 angeschlossen werden, respektive das Grüngut aus dem Container direkt an den Laderaum 21 abgegeben werden.

Die weiteren infrastrukturellen Elemente, die hier auf der Zeichnung noch erkennbar sind, sind für die Erfindung per se nicht von Bedeutung, werden aber natürlich für eine Fermentieranlage benötigt. Rein ergänzungshalber sei daher noch auf diese Elemente hingewiesen. Der Fermenter 1 wird hier über eine Austragsschnecke 8 entleert. Das fermentierte Gut wird in offenen Nachrottebunkern 35 abgelagert. Ein sogenannter Pneulader fördert das aus dem Fermenter 8 ausgetragene Material in den entsprechenden offenen Nachrottebunker 35. Die Nachrottebunker 35 werden nach einem entsprechenden Schema gefüllt und entleert. Der in den Nachrottebunkern 35 liegende Kompost kann je nach Verwendungszweck nach kürzerer oder längerer Lagerzeit weiterverwendet werden.

Im Gegensatz zu bisherigen Anlagen braucht das Frischgut somit von der Annahmestelle nicht mehr mit festen Anlagebauteilen zu einem Zerkleinerer gefördert werden und insbesondere braucht das zerkleinerte Material nicht mehr in einen siloartigen Zwischenspeicher zwischengelagert zu werden, aus dem es nachher wieder geholt werden muss, sondern wird wie bereits erwähnt praktisch auf einer Förderstrecke abgegeben mit grosser Speicherkapazität. Das Zwischenlager 20, das hier als Laderaum 21 mit Förderboden 23 und Austragseinheit 24 gestaltet ist, erlaubt praktisch, eine fliessende Zwischenlagerung mittels derer, einen solche Fermentieranlage sehr viel preiswerter gebaut werden kann.

## Patentansprüche

1. Kontinuierlich oder quasi kontinuierlich arbeitende Anlage zum Fermentieren beziehungsweise Vergären von biogenen Abfällen, die mindestens je eine Annahmeeinheit (30), ein Zwischenlager (20), eine Speiseeinheit (3) und einen Fermenter (1) umfasst, dadurch gekennzeichnet, dass das Zwischenlager (20) für die zerkleinerten biogenen Abfälle aus einem Laderaum (21) mit Förderboden (23) und einer Dosier- und Austragseinheit (24) besteht.

2. Anlage nach Patentanspruch 1, dadurch gekennzeichnet, dass die Dosier- und Austragseinheit (24) mit einer Förderstrecke (27, 28) in Verbindung steht, welche das ausgetragene Material zu der Speiseeinheit (3) fördert.

3. Anlage nach Patentanspruch 1, dadurch gekennzeichnet, dass der Laderaum (21) eine Muldenkonstruktion ist.

4. Anlage nach Patentanspruch 1, dadurch gekennzeichnet, dass der Förderboden (23) als Kratzboden oder als Endlos-Förderband oder als Moving- oder Running Floor konstruiert ist.

5. Anlage nach Patentanspruch 1, dadurch gekennzeichnet, dass die Dosier- und Austragseinheit (24) in Förderrichtung des Förderbodens (23) am Ende angeordnet ist.

6. Anlage nach den Patentansprüchen 2 und 5, dadurch gekennzeichnet, dass die Dosier- und Austragseinheit (24) ein umlaufender Kettenkratzer ist, mit dem das Material am Ende des Förderbodens (23) durch einen Austragsspalt (29) nach unten auf die Förderstrecke (27, 28) gelangt.

7. Anlage nach Patentanspruch 1, dadurch gekennzeichnet, dass die Dosier- und Austragseinheit (24) ein gegenüber dem Förderboden (23) in der Höhe verstellbar distanziertes Rückhalteelement ist.

8. Anlage nach Patentanspruch 1, dadurch gekennzeichnet, dass die Anlage einen Zerkleinerer (7) umfasst.

9. Anlage nach Patentanspruch 8, dadurch gekennzeichnet, dass der Zerkleinerer (7) ein mobiler Zerkleinerer (7) ist, der über den Laderaum bewegbar ist.

10. Anlage nach einem der Patentansprüche 1 oder 8, dadurch gekennzeichnet, dass ein Gerät (32) zum Beschicken des Zerkleinerers (7) und/oder des Laderaumes (21) mit biogenen Abfällen vorhanden ist.
